# EUROPEAN PATENT SPECIFICATION

(11) **EP 1 000 950 B1**
(45) Date of publication and mention of the grant of the patent: **25.02.2004**
(21) Application number: 99122210.0
(22) Date of filing: 06.11.1999
(51) Int. Cl.: C07F 7/08, C07F 7/21, C07C 255/41, A61K 7/42, C08G 77/388

(54) **Novel indanylidene compounds**
Indanylidenverbindungen
Composés d'indanylidène

(30) Priority: 11.11.1998 EP 98121456
(43) Date of publication of application: 17.05.2000
(73) Proprietor: F. HOFFMANN-LA ROCHE AG, 4070 Basel (CH)
(72) Inventor: Bringhen, Alain, 1244 Choulex (CH); Huber, Ulrich, 8703 Erlenbach (CH)
(74) Representative: Keller, Günter, Dr.

(56) References cited:
- EP-A- 0 823 418
- WO-A-91/16892
- WO-A-99/09112
- CHAN, JOSEPH H. ET AL: "2,4-Diamino-5-benzylpyrimidines as antibacterial agents. 14. 2,3-Dihydro-1-(2,4-diamino-5-pyrimidyl)-1H -indenes as conformationally restricted analogs of trimethoprim" J. MED. CHEM. , vol. 34, no. 2, 1991, pages 550-555, XP002053867
- BHATTACHARYYA, SUKANTA ET AL: "Selective reduction of dienones. Synthesis of intermediates for sesqui- and diterpenes" SYNTH. COMMUN., vol. 19, no. 3-4, 1989, pages 673-678, XP002053871
- BASU, BASUDEB ET AL: "Studies on intramolecular cyclizations. Synthesis of ring systems related to sesquiterpenoids" SYNTH. COMMUN. , vol. 11, no. 10, 1981, pages 803-809, XP002053870
- CROOKS, P. A. ET AL: "The synthesis and analgesic activities of some spiro[indan-1,3'- pyrrolidine] derivatives designed as rigid analogs of profadol" J. PHARM. SCI., vol. 71, no. 3, 1982, pages 291-294, XP001041834
- DAS, SWATI ET AL: "Cyclizations involving indan derivatives with aryl participation. A total synthesis of (.+-.)-isolongifolene" TETRAHEDRON LETT. , vol. 33, no. 9, 1992, pages 1229-1232, XP001041835
- CHEMICAL ABSTRACTS, vol. 104, no. 9, 3 March 1986 (1986-03-03) Columbus, Ohio, US; abstract no. 68709, SOMMERVILLE, RICHARD ET AL: "Synthesis and pharmacological evaluation of aromatic dihydroxylated spiro[indan-1,3'-pyrrolidine] and spiro[indan-2,2'-pyrrolidine] derivatives" XP002188127 & J. PHARM. SCI. (1985), 74(5), 553-5 ,

## Description

The invention relates to novel indanylidene compounds which are effective in absorbing ultra violet radiation and to light screening compositions comprising said indanylidene compounds.

Light screening compositions comprising indanylidene compounds are described in the European patent publication EP 0823 418 A2. This publication especially refers to cyano-(2,3-dihydroxy-1H-inden-1-ylidene) acetic acid ester compounds. These compounds do not have a sufficient solubility in the media usually employed in cosmetics, in particular in oil and fats. Furthermore, it is desirable that the active ingredient remain on the surface of the skin rather than penetrate into or through the skin.

It has now been found that compounds having an indanylidene residue grafted via a linker to a silane, an oligosiloxane- or a polysiloxane moiety overcome the problem of penetration and show improved solubility.

According to this invention there are provided compounds of the general formula I 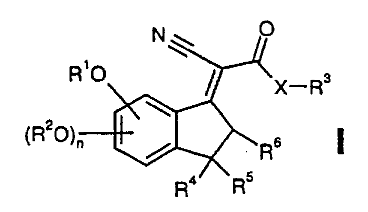 wherein
- X: signifies O or NH;
- R¹: signifies C₁-C₂₀ alkyl, C₂-C₂₀ alkyl in which at least one methylene group is replaced by oxygen, C₃-C₂₀ alkenyl, C₃-C₂₀ alkynyl or a group YS;
- R²: signifies C₁-C₂₀ alkyl, C₂-C₂₀ alkyl in which at least one methylene group is replaced by oxygen, C₃-C₂₀ alkenyl, C₃-C₂₀ alkynyl or a group YS; or R¹ and R² can combine on adjacent C-atoms to form a dioxomethylene ring;
- R³: signifies C₁-C₂₀ alkyl, C₂-C₂₀ alkyl in which at least one methylene group is replaced by oxygen, C₃-C₂₀ alkenyl, C₃-C₂₀ alkynyl or a group YS;
- R⁴,R⁵,R⁶: each independently signify H or C₁-C₂₀ alkyl;
- n: signifies 0, 1 or 2;
- Y: signifies a linker group;
- S: signifies a silane-, an oligosiloxane- or a polysiloxane-moiety;
with the proviso that at least one of the residues R¹, R² or R³ signifies YS.

The term "C₁-C₂₀ alkyl" refers in the present context to straight chain or branched saturated hydrocarbon residues with 1-20 carbon atoms such as methyl, ethyl, propyl, isopropyl, butyl, sec. butyl, isobutyl, pentyl, neopentyl, hexyl, 2-ethyl-hexyl, octyl and the like.

The term "C₂-C₂₀ alkyl in which at least one methylene group is replaced by oxygen" refers in the present context to straight chain or branched saturated hydrocarbon residues with up to 19 carbon atoms having at least a group such as -(CH₂-O)-,-(CH₂-CH₂-O)-, -(CH₂-CH₂-CH₂-CH₂-O) and the like.

The term "C₃-C₂₀ alkenyl" refers in this context to straight chain or branched unsaturated hydrocarbon residues with 3-20 carbon atoms containing a double bond such as propen-2-yl, propen-3-yl, buten-3-yl, buten-4-yl, penten-4-yl, penten-5-yl and the like.

The term "C₃-C₂₀ alkynyl" refers in this context to straight chain or branched unsaturated hydrocarbon residues with 3-20 carbon atoms containing a triple bond such as propargyl and the like.

The term "linker group" refers in this context to a C₃-C₁₂ divalent alkylene or alkenylene chain which links the silane, oligosiloxane or polysiloxane moiety to the UV absorbing chromophoric residue. The term "C₃-C₁₂ divalent alkylene chain" embraces straight chain or branched saturated hydrocarbon residues such as 3-propylene, 2-propylene, 2-methyl-3-propylene, 3-butylene, 4-butylene, 4-pentylene, 5-pentylene, 6-hexylene and the like. The term "C₃-C₁₂ divalent alkenylene chain" embraces unsaturated hydrocarbon residues containing one or multiple double bonds such as 2-propen-2-ylene, 2-propen-3-ylene, 3-buten-3-ylene 3-buten-4-ylene, 4-penten-4-ylene, 4-penten-5-ylene, (3-methyl)-penta-2,4-dien-4 or 5-ylene, 11-dodecen-l 1-ylene and the like. The chains may be interrupted by one or several oxygen atoms forming groups such as 2-ethyloxy-eth-2-ylene, 4-butyloxy-eth-2-ylene, 3,6-dioxa-8-octylene and the like. Preferred linker groups are: 3-propylene , 4-butylene, 2-propen-2-ylene, 2-propen-3-ylene or 3-buten-4-ylene.

The term "silane" refers in this context to a group -SiR⁷R⁸R⁹ wherein R⁷, R⁸ and R⁹ each independently signify C₁-C₆ alkyl, C₁-C₆ alkoxy or phenyl.

"C₁-C₆ alkyl" and "C₁-C₆ alkoxy" residues can be straight-chain or branched, such as e.g. methyl, ethyl, propyl, isopropyl, n-butyl, tert.butyl, thexyl, (1,1,2 dimethylpropyl) and, respectively, methoxy, ethoxy, propoxy, isopropoxy, n-butoxy, tert.butoxy, thexoxy. Preferred are alkyl-silane groups such as trimethylsilane, triethylsilane, tripropylsilane, triisopropylsilane, dimethyl tert.butylsilane, dimethyl thexylsilane, triphenylsilane, dimethylphenylsilane and the like.

The term "oligosiloxane" refers in this context to groups of the general formula -SiR¹⁰ₘ(OSiR¹⁰₃)ₙ with m = 0, 1 or 2; n = 3, 2 or 1 and m+n = 3; or groups of the general formula IIa or IIb 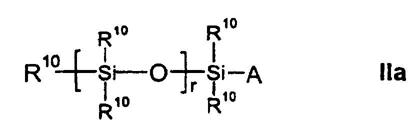 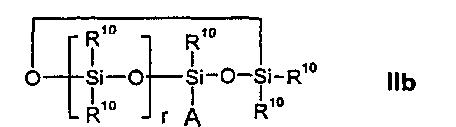 wherein
- A: signifies a bond to the linker Y;
- R¹⁰: signifies C₁-C₆ alkyl or phenyl;
- r: signifies 1 to 9.

The term "polysiloxane" refers in this context to groups of the general formulae IIIa or IIIb, 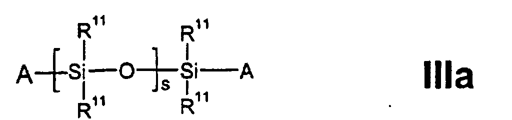 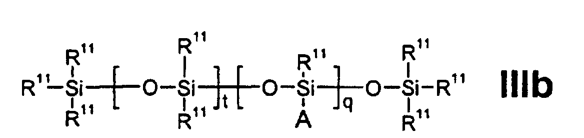 wherein
- A: is a bond to the linker Y;
- R¹¹: signifies C₁-C₆ alkyl or phenyl;
- s: has a value of from 4 to 250;
- t: has a value of from 5 to 250;
- q: has a value of from 1 to 30.

The residues R¹ and R² are preferably C₁-C₆ alkyl, more preferably C₁-C₄ alkyl, most preferably methyl, or a group YS.

The residue R³ is preferably C₁-C₆ alkyl, more preferably C₁-C₄ alkyl, most preferably ethyl, or a group YS.

The residues R⁴ and R⁵ are preferably C₁-C₆ alkyl, more preferably C₁-C₄ alkyl, most preferably methyl.

The residue R⁶ is preferably C₁-C₆ alkyl, more preferably C₁-C₄ alkyl, most preferably methyl, or hydrogen.

The residues R¹⁰ and R¹¹ are preferably C₁-C₆ alkyl, more preferably C₁-C₄ alkyl, most preferably methyl.

The value of "n" is preferably 0 or 1.

The value of "r" is preferably 1 to 3.

The value of "s" is preferably 5 to 150.

The value of "q" is preferably 2 to 10, more preferably a statistical mean value of about 4.

The value of "t" is preferably 5 to 150, more preferably a statistical mean value of about 60.

Each group R¹, R² or R³ can signify YS. Thus, the silane-, oligosiloxane- or polysiloxane moiety can be linked via Y to the indane ring or to the carboxy or amid group. Preferably the silane-, oligosiloxane- or polysiloxane moiety is linked to the carboxy or amid group (R³ is YS).

The compounds of the general formula I can be prepared as follows:

In a first step compounds of the general formula Ia 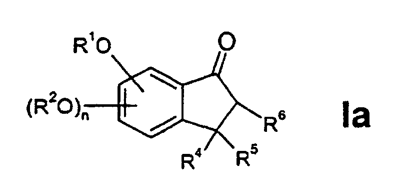 wherein R¹, R², R⁴, R⁵, R⁶ and n are as defined above are build up according to known reactions ( F. Camps, Z. Naturforsch., B : Anorg. Chem., Org. Chem. (1984), 39B (12), 1801-5), Tetrahedron Letters 27, 2941 (1973).

In a second step indanylidene compounds of the general formulae Ib, Ic and Id 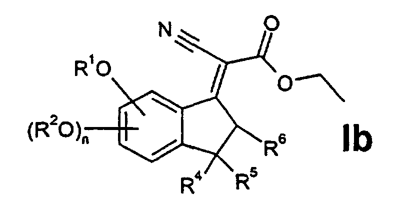 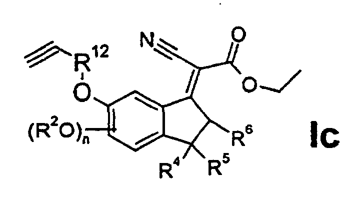 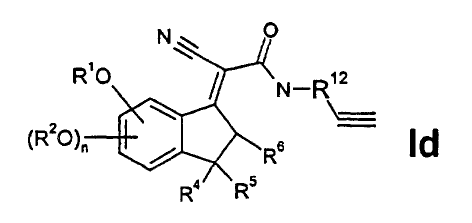 wherein R¹, R², R⁴, R⁵, R⁶ and n are as defined above and R¹² is a C₃-C₁₂ divalent alkylene- or alkenylene chain are build up according to known reactions such as the Knoevenagel reaction. As stated above the C₃-C₁₂ divalent alkylene- or alkenylene chain may be interrupted by one or several oxygen atoms.

An example to build up an indanylidene compound of the general formula Ib is given below for a compound wherein R¹ and R² are methyl, n is 1, R⁴ and R⁵ are methyl, R⁶ is hydrogen and X is oxygen. The corresponding compounds wherein R¹, R², R⁴, R⁵, R⁶ and n are as defined above can be prepared accordingly. The details are described in Example 1.

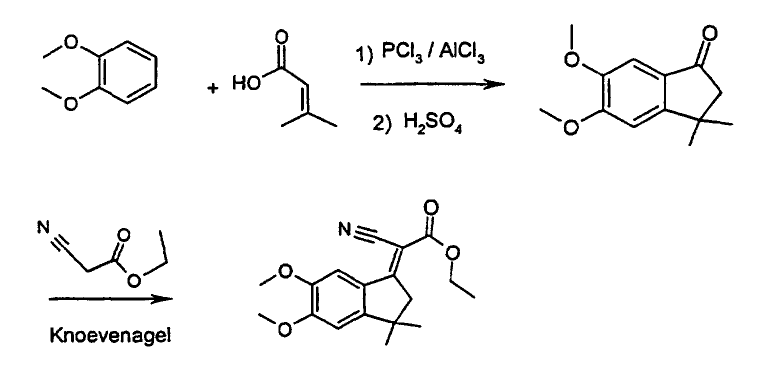

An example to build up an indanylidene compound of the general formula Ic is given below for a compound wherein R² is methyl, n is 1, R⁴ and R⁵ are methyl, R⁶ is hydrogen and X is oxygen. The corresponding compounds wherein R², R⁴, R⁵, R⁶ and n are as defined above can be prepared accordingly. The details are described in Examples 11a and 11b.

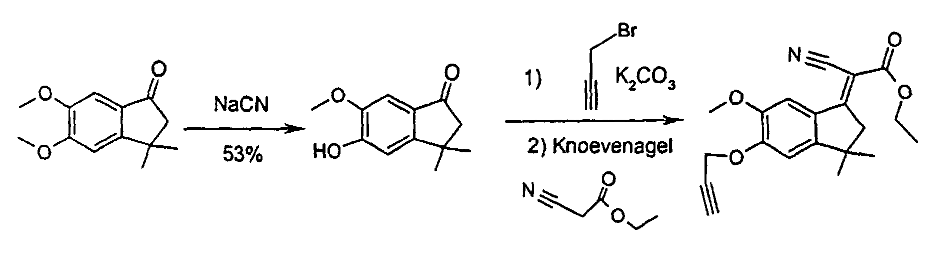

An example to build up an indanylidene compound of the general formula Id is given below for a compound wherein R' and R² are methyl, n is 1, R⁴ and R⁵ are methyl, R⁶ is hydrogen and X is nitrogen. The corresponding compounds wherein R¹, R², R⁴, R⁵ R⁶ and n are as defined above can be prepared accordingly. The details are described in Examples 8a and 8b.

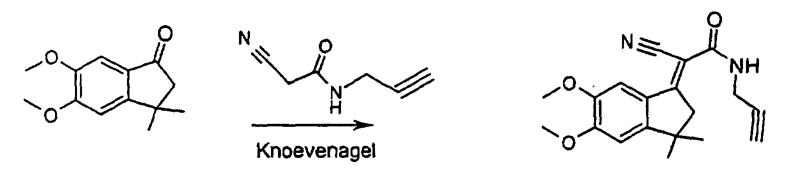

In a third step the indanylidene compounds of the general formulae Ib, Ic and Id are linked to the group YS according to known reactions either via a transesterification or a hydrosilation reaction.

An example for a transesterification reaction is given below for the reaction between a compound of the formula Ib wherein X is oxygen, R¹ and R² are methyl, n is 1, R⁴ and R⁵ are methyl, R⁶ is hydrogen and a compound ZYS, wherein Z is hydroxy, Y is 4-butyl and S is an oligosiloxane of the formula IIa wherein R¹⁰ is methyl and r is 1. The details are described in Example 2. The corresponding compounds of the general formula I wherein X is oxygen e.g. Examples 3-7 can be prepared accordingly.

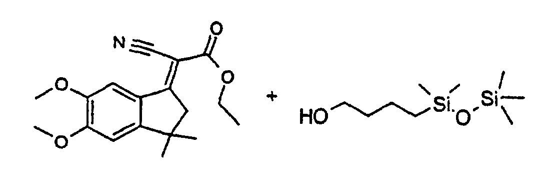

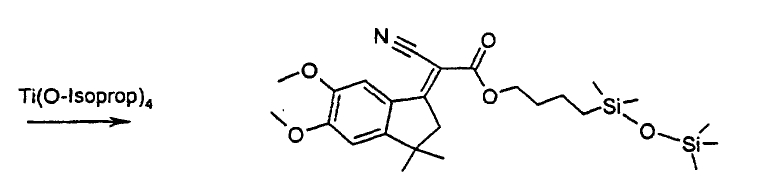

In the hydrosilation reaction compounds of the general formulae Ic and Id are reacted with a SiH containing oligosiloxane- or polysiloxane compound corresponding to the oligosiloxane- or polysiloxane residues as defined above.

Two examples for a hydrosilation reaction are given below for the reaction between a compound of the formula Id and a SiH containing oligosiloxane and polysiloxane compound respectively. The details are described in Examples 8, 9 and 10. Corresponding compounds of the general formula I wherein X is nitrogen and R³ is YS can be prepared accordingly.
a) Hydrosilation according to Example 8 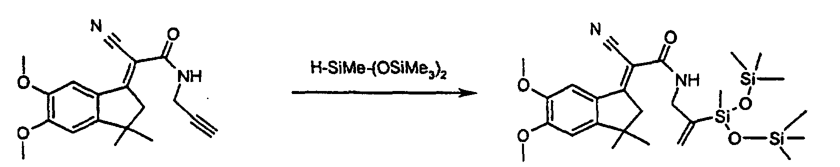
b) Hydrosilation according to Example 9

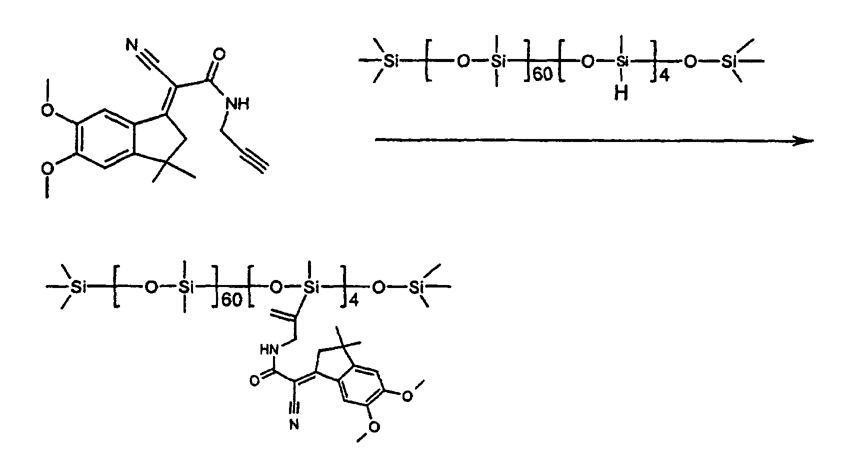

Examples 11 and 12 describe the hydrolisation reaction between a compound of the general formula Ic and a SiH containing oligosiloxane- and polysiloxane compound respectively. Compounds of the general formula I wherein X is nitrogen and R¹ is YS can be prepared accordingly.

The preparation of novel light screening agents, especially of preparations for skin protection and, respectively, sunscreen preparations for everyday cosmetics, comprises incorporating a compound of formula I in a cosmetic base which is usual for light screening agents. Where convenient, other conventional UV-A, and respectively, UV-B filters can also be combined during this incorporation. Said combinations of UV filters can show a synergistic effect. The preparation of said light screening agents is well known to the skilled artisan in this field. The amount of compounds of the general formula I and other known UV-filters is not critical. Suitable amounts are about 0.5 to about 12%.

Suitable UV B filters, i.e. substances having absorption maxima between about 290 and 320 nm, are for example the following organic compounds which belong to the widest classes of substance:
--- p-Aminobenzoic acid derivatives such as ethyl, propyl, butyl, isobutyl, octyldimethyl, amyldimethyl, ethoxylated ethyl, propoxylated ethylglyceryl or ethylglycosyl p-aminobenzoate and the like;
--- Acrylates such as 2-ethylhexyl 2-cyano-3,3-diphenylacrylate (octocrylene), ethyl 2-cyano-3,3-diphenylacrylate and the like;
--- Aniline derivatives such as methyl anilinum methosulfate and the like;
--- Anthranilic acid derivatives such as menthyl anthranilate and the like;
--- Benzophenone derivatives such as benzophenone-1 to benzophenone-12 and the like.
--- Camphor derivatives such as methyl benzylidene camphor, 3-benzylidene camphor, camphor benzalkonium methosulfate, polyacrylamidomethyl benzylidene camphor, sulfo benzylidene camphor, sulfomethylbenzylidene camphor, therephthalidene dicamphor sulfonic acid and the like;
--- Cinnamate derivatives such as octyl methoxycinnamate or ethoxyethyl methoxycinnamate, diethanolamine methoxycinnamate, isoamyl methoxycinnamate and the like as well as cinnamic acid derivatives bond to siloxanes;
--- Gallic acid such as digalloyl trioleate and the like;
--- Imidazole derivatives such as e.g. phenyl benzimidazole sulfonic acid and their salts;
--- Salicylate derivatives such as isopropylbenzyl, benzyl, butyl, octyl, isooctyl or homomenthyl salicylate and the like;
--- Triazole derivatives such as drometriazole, hydroxydibutylphenyl-, hydroxydiamylphenyl-, hydroxyoctylphenyl- or hydroxyphenylbenztriazole and the like;
--- Triazone derivatives such as octyl triazone, dioctyl butamidotriazone and the like; and
--- Pigments such as e.g. microparticulated TiO₂.

The formulation may further contain UV-A filters such as
--- Dibenzoylmethane derivatives such as 4-tert. butyl-4'-methoxydibenzoyl-methane and the like;
--- Pigments such as e.g. microparticulated ZnO..
--- Triazine compounds as described in the European Patent Publications EP 0693483 Al, EP 0704437 A2, EP 0704444 Al and EP 0780382 A1;
--- Organosiloxane compounds as described in the European Patent Publications EP 0538431 B1, EP 0709080 A1 and EP 0358584B1;
--- Malonates such as described in the European Patent Publication EP 895776 A2.

The term "microparticulated" refers to a particle size from about 5 nm to about 200 nm, particularly from about 15 nm to about 100 nm.

As cosmetic bases usual for light screening compositions in the scope of the present invention there can be used any conventional preparation which corresponds to the cosmetic requirements, e.g. creams, lotions, emulsions, salves, gels, solutions, sprays, sticks and milks; see also, Sunscreens, Development, Evaluation and Regulatory Aspects, ed. N.Y. Lowe, N.A. Shaath, Marcel Dekker, Inc. New York and Basel, 1990.

Having regard to their good lipophility, the compounds of the general formula I can be incorporated well into oil containing and fat containing cosmetic preparations such as e.g. in cosmetic preparations containing dimethicone.

The following examples illustrate the invention in more detail, but do not limit its scope in any manner.

### Example 1:

Cyano-(2,3-dihydro-5,6-dimethoxy-3,3-dimethyl -1H-inden-1-ylidene) acetic acid ethyl ester

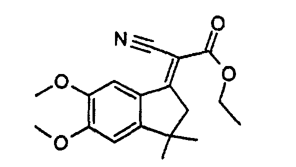

122 g (0.55 mol) of 2,3-Dihydro-5,6-dimethoxy-3,3-dimethyl-1H-inden-1-one (CAS N°[4136-26-9] for preparation see F. Camps, Z. Naturforsch., B : Anorg. Chem., Org. Chem. (1984), 39B (12), 1801-5), 63,3 g (0.56 mol) of ethyl cyano acetate, 12 g (0.14 mol) of piperidine, 12 g (0.1 mol) of benzoic acid and 1000 ml toluene were mixed together and heated under reflux for 48 hours. The reaction mixture was cooled to room temperature, washed with diluted HCl, aq Na₂CO₃, water and dried (MgSO₄). Removal of the solvent and crystallization from EtOH gave 123.8 g of a yellow solid (m.p. : 148-149 °C). Yield : 71%; E^{1%}cm: 790 (1 max. : 367 nm) in EtOH. Solubility: 0.35% in Cétiol LC

### Example 2:

Cyano-(2,3-dihydro-5,6-dimethoxy-3,3-dimethyl -1H-inden-1-ylidene) acetic acid-3-(1,1,3,3,3-pentamethy-disiloxanyl)-propyl ester

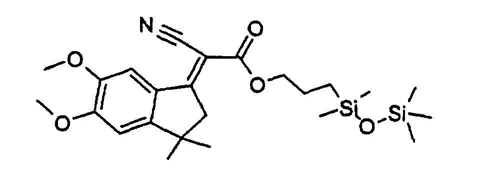

### a) 3-(1,1,3,3,3-Pentamethy-disiloxanyl)-propanol

A 50 ml reaction flask was charged with 13.6 ml (200 mmol) of allylic alcohol and a catalytic amount of divinyl-tetramethyl disiloxane platinum complex under inert atmosphere and heated to 60°C. 19.5 ml (100mmol) of pentamethyl disiloxane was slowly added through a dropping funnel. The exothermic reaction mixture was stirred over night at 75°C, followed by a distillation at 105 to 107°C / 40 to 41 mbar over a Vigreux column. Yield 18.3 g (88.5% of the theory) of a clear liquid.

### b) Transesterification

A 25 ml reaction flask equipped with a distillation bridge and connected to vacuum was charged with 2 g (6.3 mmol) of cyano-(2,3-dihydro-5,6-dimethoxy-3,3-dimethyl-1H-inden-1-ylidene) acetic acid ethyl ester (see Example 1), 1.37 g (6.6 mmol) of 3-(1,1,3,3,3-pentamethy-disiloxanyl)-propanol (see above) and 3 mg of tetraisopropyl ortho titanate. The mixture was heated to 115 to 120°C at a vacuum of 360 mbar with stirring for 11 hours. The excess of silylated alcohol was removed at 100°C /0.2 mbar and the residual product was chromatographed over SiO₂ in hexane: ethylacetate (9.1 to 8:2) to yield 1.2 g (41%) of a liquid honey (m.p.: ca. 25°C), UV 364 nm (ε=26'093), which showed excellent solubility in cosmetic solvents such as >20% in Cétiol LC (Coco-caprylate/caprate) and was miscible in Crodamol DA (Diisopropyl adipate). The product showed an excellent photostability in high dilution of an ethanol solution using a Hg-lamp 150 W from Heraeus with Pyrex filter.

### Example 3:

Cyano-(2,3-dihydro-5,6-dimethoxy-3,3-dimethyl -1H-inden-1-ylidene) acetic acid-3-(1,1,3,3,3-pentamethy-disiloxanyl)-2-methyl-propyl ester

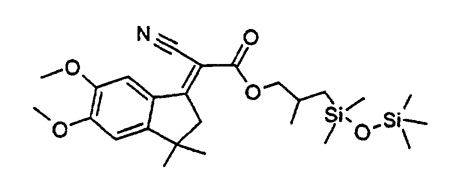

### a) 3-(1,1,3,3,3-Pentamethy-disiloxanyl)-2-methyl propanol

The reaction of Example 2a was repeated using 2-methallyl alcohol instead of allyl alcohol. After distillation at 105°C / 40 x 10² Pa, 81% of a clear liquid product was obtained.

### b) Transesterification

The reaction of Example 2b was repeated using 3-(1,1,3,3,3-pentamethydisiloxanyl)-2-methyl propanol (see above) instead of 3-(1,1,3,3,3-pentamethydisiloxanyl)-propanol. After 15 hours reaction time the product was concentrated and chromatographed as above to yield 57% of a liquid material. UV 364 nm (ε=25'200), having the same solubility and photostability qualities as described in Example 2b.

### Example 4:

Cyano-(2,3-dihydro-5,6-dimethoxy-3,3-dimethyl -1H-inden-1-ylidene) acetic acid-4-(1,3,3,3-tetramethyl-1-[(trimethyl silyl)-oxy]-disiloxanyl)-butyl ester

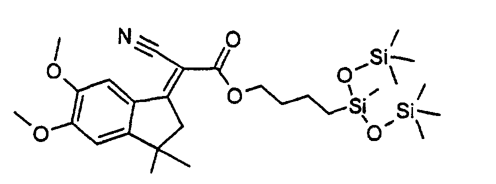

### a) 4-(1,3,3,3-Tetramethyl-1-[(trimethyl silyl)-oxy]-disiloxanyl)-butanal

The reaction of Example 2a was repeated using 1,1,1,3,5,5,5-heptamethyl trisiloxane instead of 1,1,3,,3,3-pentamethyl disiloxane and 3-butenol instead of allylalcohol. After distillation at 78°C / 0.1 x 10² Pa, 83% of a clear liquid product was obtained.

### b) Transesterification

A 25 ml reaction flask equipped with a distillation bridge and connected to vacuum was charged with 1.67 g (5.3 mmol) of Cyano-(2,3-dihydro-5,6-dimethoxy-3,3-dimethyl-1H-inden-1-ylidene) acetic acid ethyl ester (see example 1), 1.64 g (5.6 mmol) of 4-(1,3,3,3-tetramethyl-1-[(trimethyl silyl)-oxy]-disiloxanyl)-butanol (see above) and 3 mg of tetraisopropyl ortho titanate. The mixture was heated to 125°C at a vacuum of 270 mbar under stirring for 9 hours. The excess of silylated alcohol was removed at 0.2 mbar and the residual product was chromatographed over SiO₂ in hexane: ethylacetate (9.1 to 7:3) to yield 2.3 g (78%) of a liquid, UV 364 nm (ε=25'800) and 376 nm (ε=25'180). The product was miscible in Cétiol LC and Crodamol DA and showed an excellent photostability in high dilution of an ethanol solution using a Hg-lamp 150 W from Heraeus with Pyrex filter.

### Example 5:

Cyano-(2,3-dihydro-5,6-dimethoxy-3,3-dimethyl -1H-inden-1-ylidene) acetic acid-4-(2-triethylsilanyl-ethoxy)-butyl ester

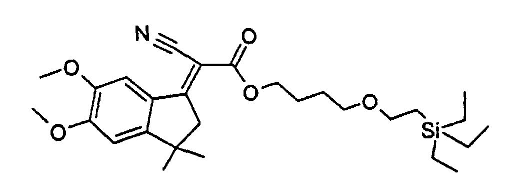

### a) 4-(2-Triethylsilanyl-ethoxy)-butanol

A 50 ml reaction flask was charged with 11.6 ml (100 mmol) of 1,4-butandiol-mono vinylether and a catalytic amount of divinyl-tetramethyl disiloxane platinum complex under inert atmosphere and heated to 60°C. 10.4 g (90 mmol) of triethylsilane was slowly added through a dropping funnel. The exothermic reaction mixture was stirred at 75°C for 18 hours, followed by distillation at 105 to 107°C / 0.2 mbar over a 10 cm Vigreux column. Yield 15.2 g (66% of the theory) of a clear liquid. Purity: 98.7% according to gas chromatography.

### b) Transesterification

The reaction of Example 2b was repeated using 4-(2-triethylsilanyl-ethoxy)-butanol (see above) instead of 3-(1,1,3,3,3-pentamethy-disiloxanyl)-propanol. After 8 hours reaction time the product was concentrated and chromatographed as above to yield 70% of a liquid material. UV 364 nm (ε=27'126), having the same solubility and photostability qualities as described in Example 2b.

### Example 6:

Cyano-(2,3-dihydro-5,6-dimethoxy-3,3-dimethyl -1H-inden-1-ylidene) acetic acid-4-triethylsilanyl-but-3-enyl ester

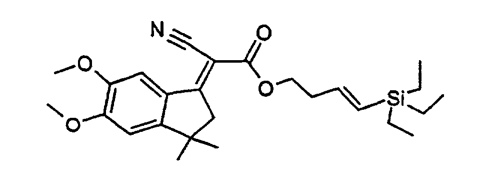

### a) 4-Triethylsilanyl-1-but-3-enol

A 50 ml reaction flask was charged with 1-butyne-3-ol and a catalytic amount of bis(1,5-ryclooctadiene)-di-Rh(I)-dichloride and triphenylphosphine under inert atmosphere. Triethylsilane was slowly added through a dropping funnel. The reaction mixture was stirred at room temperature for 72 h and then concentrated at the rotary evaporator. The product was chromatographed through SiO₂ in hexane : ethylacetate (95:5 to 70:30) to yield 86% of a yellow liquid. Purity according to gas chromatography: 96% trans and 3.4% cis product.

### b) Transesterification

The reaction of Example 2b was repeated using 4-triethylsilanyl-1-but-3-enol (see above) instead of 3-(1,1,3,3,3-pentamethy-disiloxanyl)-propanol. After 9 hours reaction time the product was concentrated and chromatographed as above to yield 65% of a semicrystalline material. UV 368 nm (ε=26'748), having the same solubility and photostability qualities as described in Example 2b.

### Example 7:

Cyano-(2,3-dihydro-5-methoxy-2-methyl -1H-inden-1-ylidene) acetic acid-4-(1,1,3,3,3-pentamethy-disiloxanyl)-butyl ester 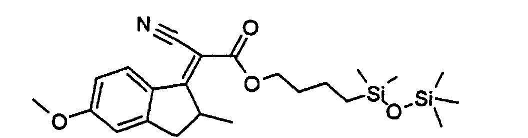
a) 4-( 1,1,3,3,3-Pentamethy-disiloxanyl)-butanol
b) A 50 ml reaction flask was charged with 10.3 ml of 3-butene-1-01 and a catalytic amount of divinyl-tetramethyl disiloxane platinum complex under inert atmosphere and heated to 60°C. 19.5 ml of pentamethyl disiloxane was slowly added through a dropping funnel. The reaction mixture was stirred at 75 to 80°C for three hours, followed by distillation at 110 to 115°C / 38 x 10² Pa over a 10 cm column. Yield: 18.9 g (86% of the theory) of a clear liquid.
c) 2,3-Dihydro-5-methoxy-2-methyl-1H-inden-1-one
   5-Methoxy indanone was treated with aqueous formaldehyde in the presence of iron pentacarbonyl and KOH in ethanol according to G. Cainelli et. al., *Tetrahedron Letters 27,* 2491 (1973). After chromatography (hexane: ethylacetate = 7:3) over SiO₂ 44% yield of white crystals are obtained (m.p. 73-76°C).
c) Cyano-(2,3-dihydro-5-methoxy-2-methyl -1H-inden-1-ylidene) acetic acid 2-ethyl-hexyl ester
   The above 2,3-dihydro-5-methoxy-2-methyl-1H-inden-1-one (5.2 g) was reacted with 5.9 g of 2-ethyl-hexyl cyano acetate in the presence of catalytic amounts of pyrrolidine and benzoic acid in 100 ml of toluene. The reaction mixture was refluxed for 30 hours with simultaneous separation of water. Then the cold reaction mixture was washed with water, concentrated and chromatographed in toluene containing 2% of propanol through SiO₂ to yield 2.6 g of a yellow liquid. UV 347 nm (ε=33'450), MS: 355(M⁺), 243 (100%), 226, 198.
d) Transesterification

The reaction of Example 2b was repeated using 4-(1,1,3,3,3-pentamethydisiloxanyl)-butanol (see above) instead of 3-(1,1,3,3,3-pentamethy-disiloxanyl)-propanol as well as the above cyano-(2,3-dihydro-5-methoxy-2-methyl -1H-inden-1-ylidene) acetic acid 2-ethyl-hexyl ester instead of the product of example 1. After 9 hours reaction time the product was concentrated and then treated again for 5 hours as above with new 3-(1,1,3,3,3-pentamethy-disiloxanyl)-propanol. Now the mixture was concentrated and chromatographed as before to yield 30% of a liquid material. UV 347 nm (ε=32'500), having the same solubility and photostability qualities as described in Example 2b.

### Example 8:

Cyano-(2,3-dihydro-5,6-dimethoxy-3,3-dimethyl -1H-inden-1-ylidene) acetic acid-2-(1,3,3,3-tetramethyl-1-[(trimethyl silyl)-oxy]-disiloxanyl)-prop-2-enyl amide

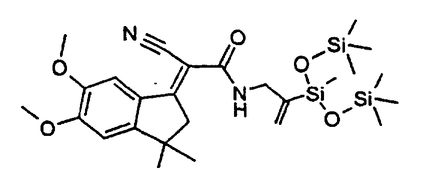

### a) 2-Cyano-N-prop-2-ynyl-acetamide

A mixture of 15.4 ml (240 mmol) of propargyl amine and 17.1 ml (160 mmol) of ethyl cyano acetate was heated for 7 hours to 40°C in a reaction flask. The crystalline material formed was heated further for 17 hours to 70°C. Then the cooled reddish product was dried at high vacuum to yield 18.4 g (94%) of a red powder, m.p. 100-103°C.

### b) 2-Cyano-2-(5,6-dimethoxy-3,3-dimethyl-indan-1-ylidene)-N-prop-2-ynylacetamide

In a 50 ml reaction flask equipped with a water separator and a reflux condenser was charged with 2.2 g of 2,3-dihydro-5,6-dimethoxy-3,3-dimethyl-1H-inden-1-one (see Example 1) and 1.2 g of 2-cyano-N-prop-2-ynyl-acetamide (see above), catalytic amounts of pyrrolidine and benzoic acid in 20 ml of toluene. The reaction mixture was refluxed for 24 hours with simultaneous separation of water. Then the product in the cold reaction mixture was filtered off and recrystallized in ethylacetate to yield 1.13 g of white crystals, m.p. 202-205°C. UV 362 nm (ε=24'992).Solubility :0.04% in Cétiol LC

### c) Hydrosilylation reaction

The above 2-cyano-2-(5,6-dimethoxy-3,3-dimethyl-indan-1-ylidene)-N-prop-2-ynyl-acetamide (320 mg), 220 mg of 1,1,1,3,5,5,5-heptamethyl trisiloxane and a catalytic amount of divinyl-tetramethyl disiloxane platinum complex in 10 ml of toluene was placed in a three-necked reaction flask under inert atmosphere and stirred for 48 hours at 95°C. The product solution is washed with a mixture of water/methanol = 1:10 and concentrated to yield 550 mg (100%) of a yellow liquid. UV 360 nm (ε=22'069), MS: 546 (M⁺), 531, 299, 270, 269 (100%). Its NMR shows a mixture of the vicinal and the geminal hydrosilylation product of 1:2. The product was miscible in CETIOL LC and CRODAMOL DA and showed the same photostability qualities as described in Example 2b.

### Example 9

A polysiloxane which corresponds in its statistical mean value to the following formula:

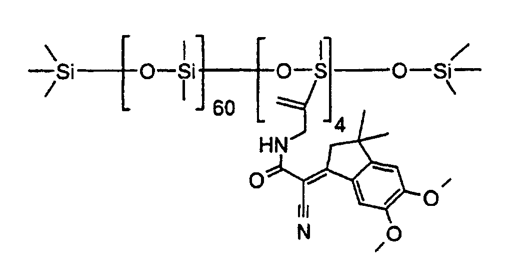

2-Cyano-2-(5,6-dimethoxy-3,3-dimethyl-indan-1-ylidene)-N-prop-2-ynylacetamide (320 mg), 1180 mg of polysiloxane Ae-151 of Wacker-Chemie GmbH. and a catalytic amount of divinyl-tetramethyl disiloxane platinum complex in 10 ml of toluene was placed in a three-necked reaction flask under inert atmosphere and heated for 48 hours to 95°C. The product solution was washed with a mixture of water/methanol = 1:10 and concentrated to yield 1500 mg (100%) of a yellow liquid. UV 360 nm (E=210.6). The product was miscible in CETIOL LC and CRODAMOL DA and showed the same photostability qualities as described in Example 2b.

### Example 10

α-(Dimethyl-[2N-[2-cyano-2-(5,6-dimethoxy-3,3-dimethyl-indan-1-ylidene)-acetamide]-1-methylene-ethyl ]-ω-(dimethyl-[2N-[2-cyano-2-(5,6-dimethoxy-3,3-dimethyl-indan-1-ylidene)-acetamide)-1-methylene-ethyl]-poly-(oxy-(dimethyl)-silene), n~9

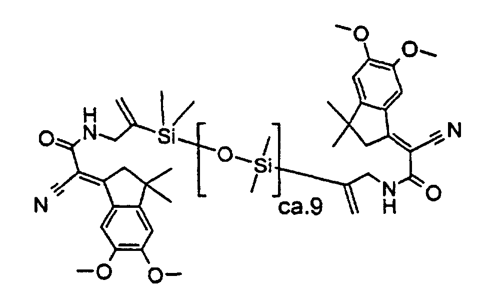

2-Cyano-2-(5,6-dimethoxy-3,3-dimethyl-indan-1-ylidene)-N-prop-2-ynylacetamide (320 mg), 340 mg of polysiloxane VP-1085 of Wacker-Chemie GmbH. and a catalytic amount of Pt on charcoal 5% in 10 ml of toluene was placed in a three-necked reaction flask under inert atmosphere and heated for 44 hours to 105°C. The product solution was filtered through Cellite, washed with a mixture of water/methanol = 1:10 and concentrated to yield 560 mg (85%) of a yellow liquid. UV 360 nm (E=329). The product was miscible in CETIOL LC and CRODAMOL DA and showed the same photostability qualities as described in Example 2b.

### Example 11

Cyano-(6-methoxy-3,3-dimethyl-5-[2-((1,3,3,3-tetramethyl-1-[(trimethyl silyl)-oxy]-disiloxanyl)-allyloxy]-indan-1-ylidene)-acetic acid ethyl ester

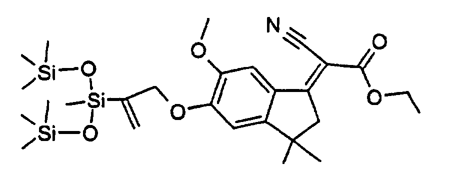

### a) 6-Methoxy-3,3-dimethyl-5-hydroxy-indan-1-one

In a three necked reaction flask equipped with a reflux condenser, a mixture of 5 g of 2,3-dihydro-5,6-dimethoxy-3,3-dimethyl-1H-inden-1-one (see example 1) and 5.5 g of sodium cyanide in 44.5 ml of dimethyl formamide was stirred at 100°C for 40 hours. Then the mixture was pored on an aqueous NaH₂PO₄ solution and extracted 4 times with ethyl acetate, dried over Na₂CO₃ and concentrated. The raw product was chromatographed in methylene chloride containing 1% of methanol to yield 2.5 g of white crystals. m.p. 105-107°C. MS: 206(M⁺), 191(100%), 163, 131, 103.

### b) 6-Methoxy-3,3-dimethyl-5-prop-2-ynyloxy-indan-1-one

6-Methoxy-3,3-dimethyl-5-hydroxy-indan-1-one (2.2 g), 1.5 g of propargyl bromide and 3.7 g of K₂CO₃ in 9 ml of 1-methyl pyrrolidone was placed in a 25 ml reaction flask and stirred for one hour. Then it was stirred further at 100°C for one hour. The reaction mixture was distributed between water and ethyl acetate. The organic phase was washed with In NaOH solution and NaCl solution, dried over Na₂SO₄ and concentrated to yield 2.7 g of a yellow liquid. UV 310 nm (ε=18'734), MS: 244(M⁺), 229,105(100%).

### c) Cyano-(6-methoxy-3,3-dimethyl-5-prop-2-ynyloxy-indan-1-ylidene)-acetic acid ethyl ester

The above 6-methoxy-3,3-dimethyl-5-prop-2-ynyloxy-indan-1-one (2.44 g) was treated with 1.13 g of ethyl cyano acetate in the presence of 0.1 equivalents of pyrrolidine and benzoic acid in 20 ml of xylene. The reaction mixture was refluxed for 30 hours with simultaneous separation of water. Then the cold reaction mixture was washed with water, concentrated and chromatographed in hexane / ethyl acetate through SiO₂ to yield 0.7 g of yellow crystals. m.p. 132-136°C. UV 362 nm (ε=23'222), MS: 339(M⁺), 324, 300 (100%).

### d) Hydrosilylation reaction

Cyano-(6-methoxy-3,3-dimethyl-5-prop-2-ynyloxy-indan-1 -ylidene)-acetic acid ethyl ester (500 mg), 330 mg of 1,1,1,3,5,5,5-heptamethyl trisiloxane and a catalytic amount of divinyl-tetramethyl disiloxane platinum complex in 15 ml of toluene was placed in a three-necked reaction flask under inert atmosphere and heated for 24 hours to 78°C. The product solution was concentrated and filtered through SiO₂ in hexane / ethyl acetate = 9:1 and again concentrated to yield 590 mg (71%) of a yellow liquid. UV 367 nm (ε=23'515), MS: 561(M⁺), 546, 509(100%). Its NMR shows a mixture of the vicinal and the geminal hydrosilylation product of 2:1. The product was miscible in CETIOL LC and CRODAMOL DA and showed the same photostability qualities as described in Example 2b.

### Example 12

A polysiloxane which corresponds in its statistical mean value to the following formula:

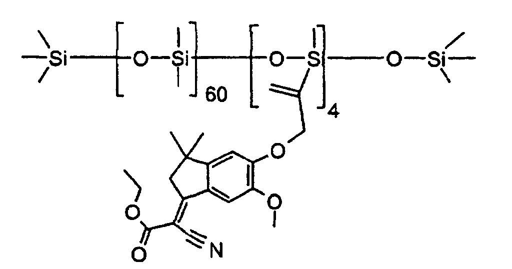

Cyano-(6-methoxy-3,3-dimethyl-5-prop-2-ynyloxy-indan-1-ylidene)-acetic acid ethyl ester (280 mg), 770 mg of polysiloxane Ae-151 of Wacker-Chemie GmbH. and a catalytic amount of divinyl-tetramethyl disiloxane platinum complex in 10 ml of toluene was placed in a three-necked reaction flask under inert atmosphere and heated for 20 hours to 80°C. The product solution was washed with a mixture of water/methanol =1:10, concentrated and filtered through SiO₂ to yield 1100 mg (100%) of a yellow liquid. UV 366 nm (ε=26'172 / E= 180.5). Its NMR shows both vicinal and geminal hydrosilylation product. The product was miscible in CETIOL LC and CRODAMOL DA and shows the same photostability qualities as described in Example 2b.

### Example 13

A polysiloxane which corresponds in its statistical mean value to the following formula

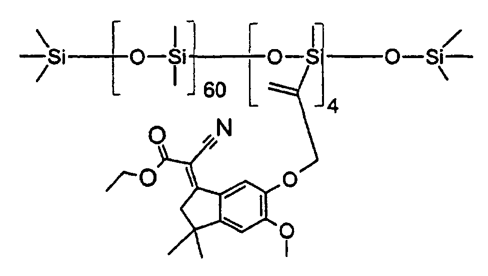

In a first step 5-methoxy-3,3-dimethyl-6-prop-2-ynyloxy -indan-1-one was prepared as follows:

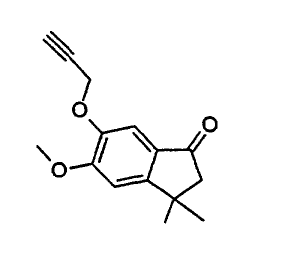

45.8 g of 5-methoxy-3,3-dimethyl-6-hydroxy-indan-1-one (CAS N°[98910-58-8] 24.4 g of propargyl chloride and 8.9 g of NaH in 300 ml of DMF were stirred for one hour at room temperature and then heated for 20 hours at 50°C. The reaction mixture was distributed between water and toluene. The organic phase was washed with Na₂CO₃ and water, dried over Na₂SO₄ and concentrated to yield 41.7 g of a brown liquid. ¹H-NMR (200 MHz): 1.41 (s, 6H); 2.52-2.58 (m, 3H); 3.99 (s, 3H); 4.79 (d, J= 2 Hz, 2H); 6.89 (s, 1H); 7.27 (s, 1H).

To prepare cyano-(5-methoxy-3,3-dimethyl-6-prop-2-ynyloxy-indan-1-ylidene)-acetic acid ethyl ester

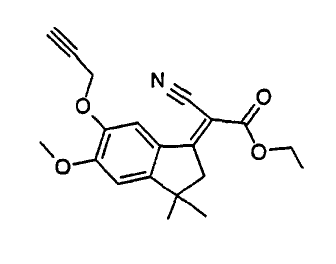

41.7 g of the above 5-methoxy-3,3-dimethyl-6-prop-2-ynyloxy-indan-1-one was treated with 21.49 g of ethyl cyano acetate in the presence of 16 g of piperidine and 16 g of benzoic acid in 1300 ml of cyclohexane. The reaction mixture was refluxed for 24 hours with simultaneous separation of water. Then the cold reaction mixture was washed with water, HCl 1%, Na₂CO₃ and water. The reaction mixture was concentrated and recristallised in EtOH to yield 24.3 g of yellow crystals.. ¹H-NMR (200 MHz): 1.33 (s, 6H); 1.38 (t, J= 7Hz, 3H); 2.57 (t, J= 2 Hz, 1H); 3.37 (s, 3H); 3.99 (s, 3H); 4.31 (q, J= 7Hz, 2H); 4.83 (d, J= 2 Hz, 2H); 6.80 (s, 1H); 8.26 (s, 1H).

α-(Trimethylsilyl)-ω-(trimethylsilyl-oxy)-poly-(oxy-(dimethyl)- and ca. 7.5% of methyl-(6-[-1-cyano-ethyloxy-acetyl-(5-methoxy-3,3-dimethyl-indan-1-ylidene]-1-methylene-eth-2-oxy)-silene)

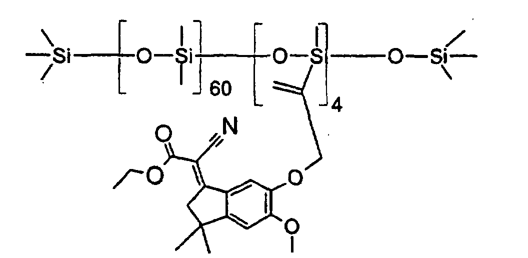

16.13 g of the above Cyano-(5-methoxy-3,3-dimethyl-6-prop-2-ynyloxy-indan-1-ylidene)-acetic acid ethyl ester, 54.95 g of polysiloxane Ae-151 of Wacker-Chemie GmbH. and a catalytic amount of Pt/C (Heraeus type k-0101) in 75 ml of toluene were heated for 28 hours at 110°C. The reaction mixture was filtrated and then washed with a mixture of water/methanol = 1:5, concentrated to give 67.8 g a yellow liquid. UV 368 nm (E=150). Its NMR shows both vicinal and geminal hydrosilylation product. The product was miscible in CETIOL LC and CRODAMOL DA and shows the same photostability qualities as described in Example 2b.

### Solubility

The compounds of the general formula I are excellent soluble in cosmetic solvents. The compounds of Examples 2 to 12 are miscible in CRODAMOL DA. The solubility in CETIOL LC is >20% for compounds of Examples 2, 3, 5 and 6. Examples 4 and 8 to 12 are miscible in CETIOL LC.

The following Table 1 shows solubility data of indanylidene compounds disclosed in the European patent publication EP 0823 418 A2 and of compounds according to the invention.

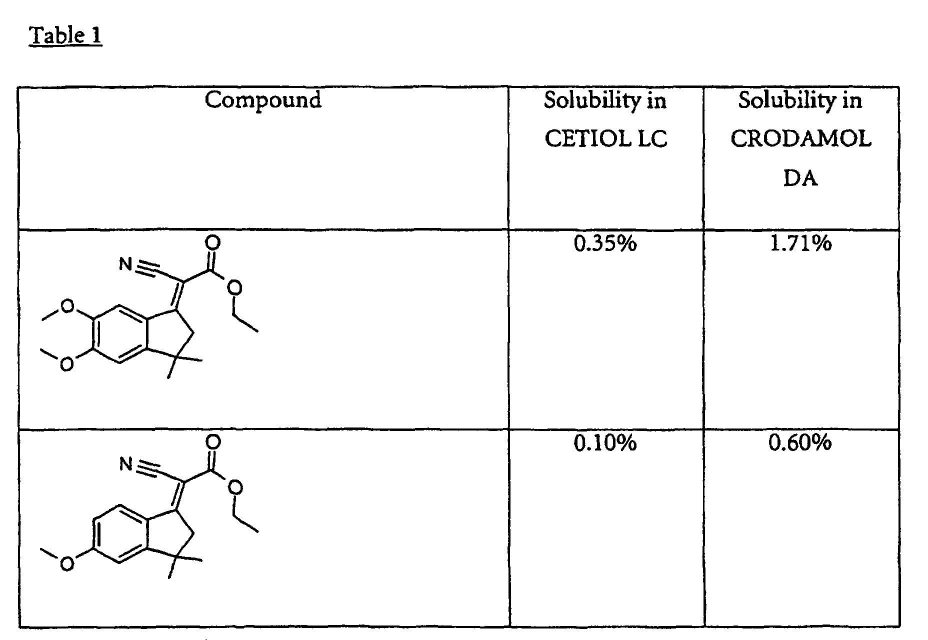

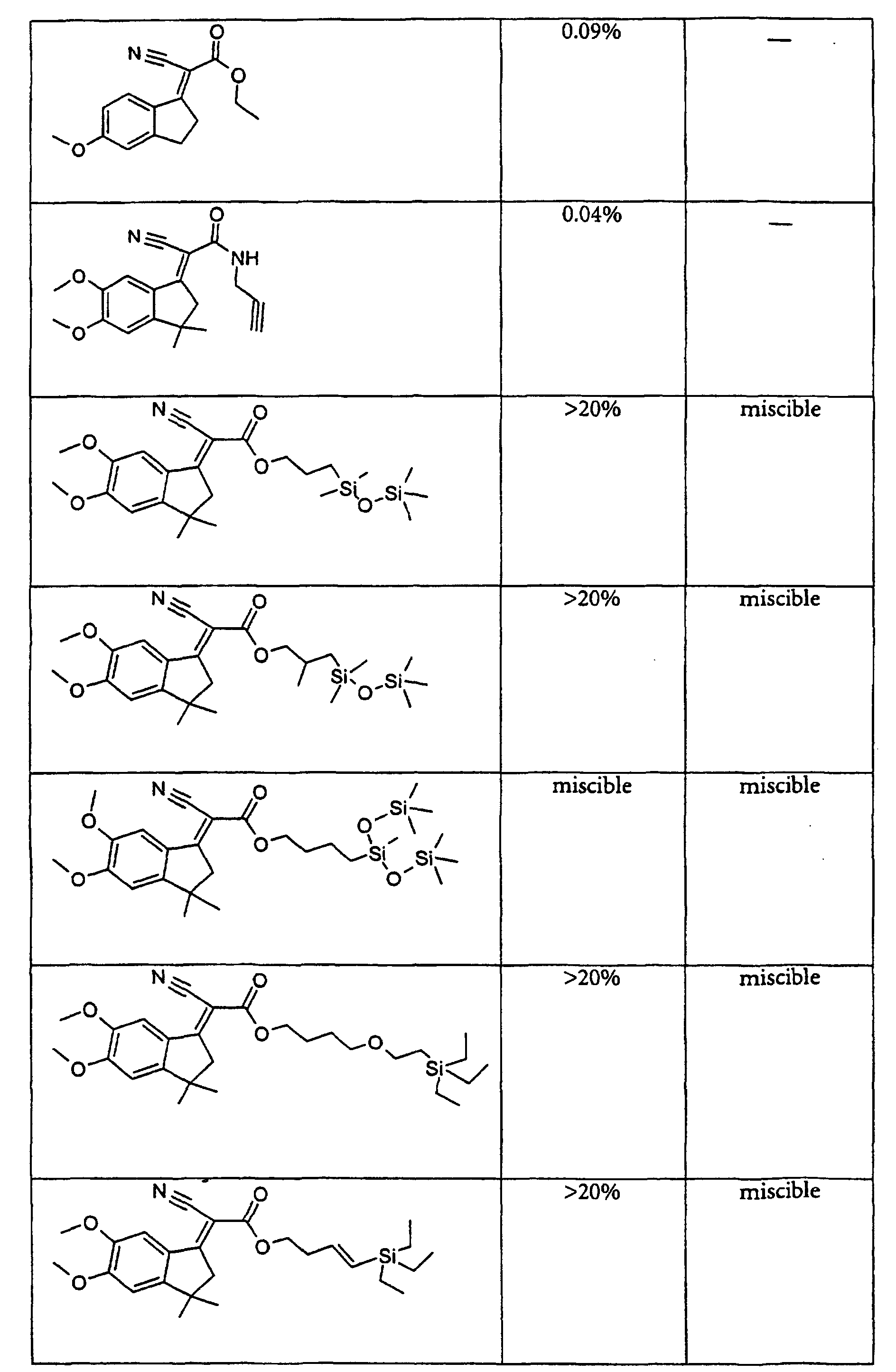

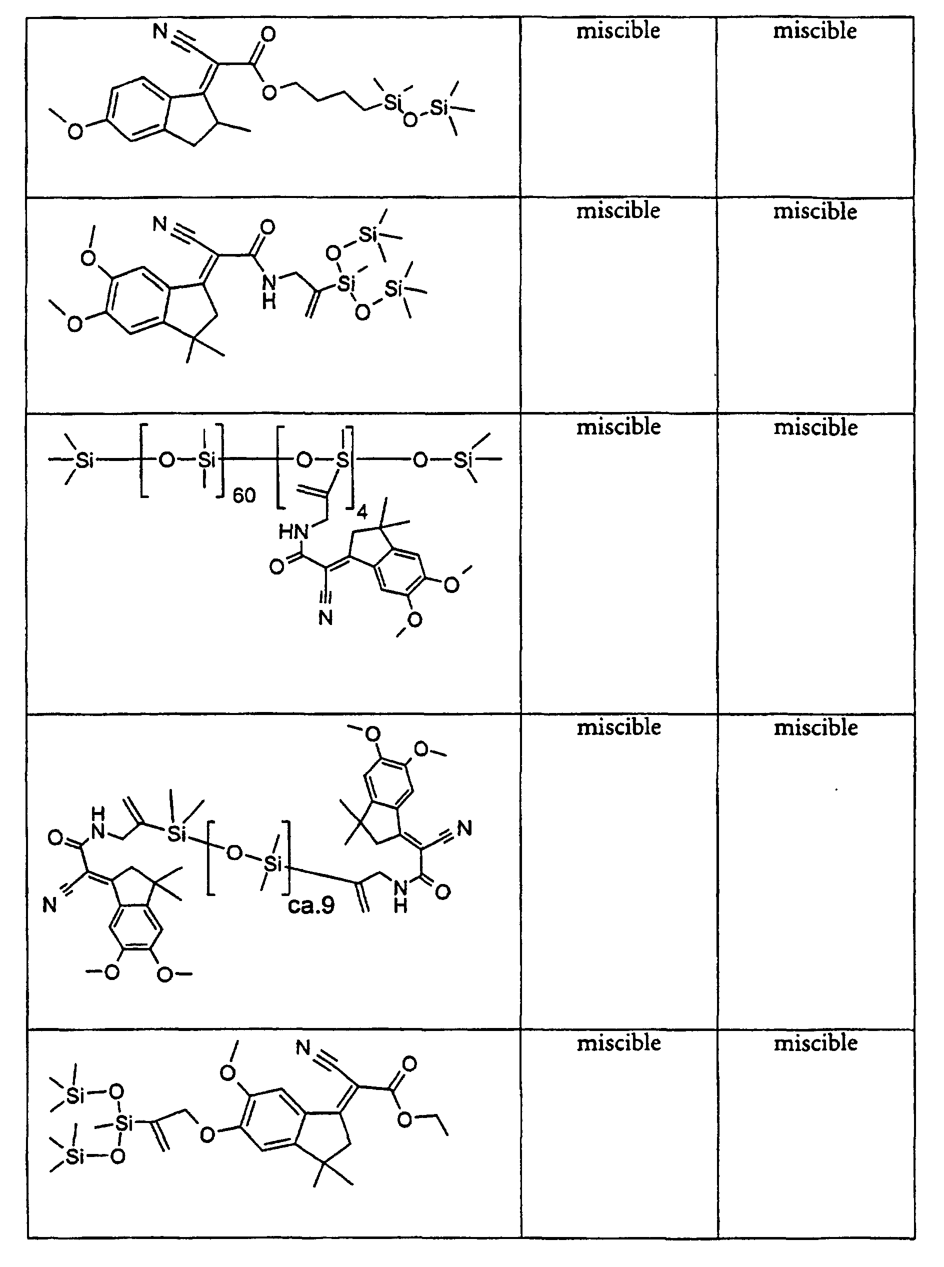

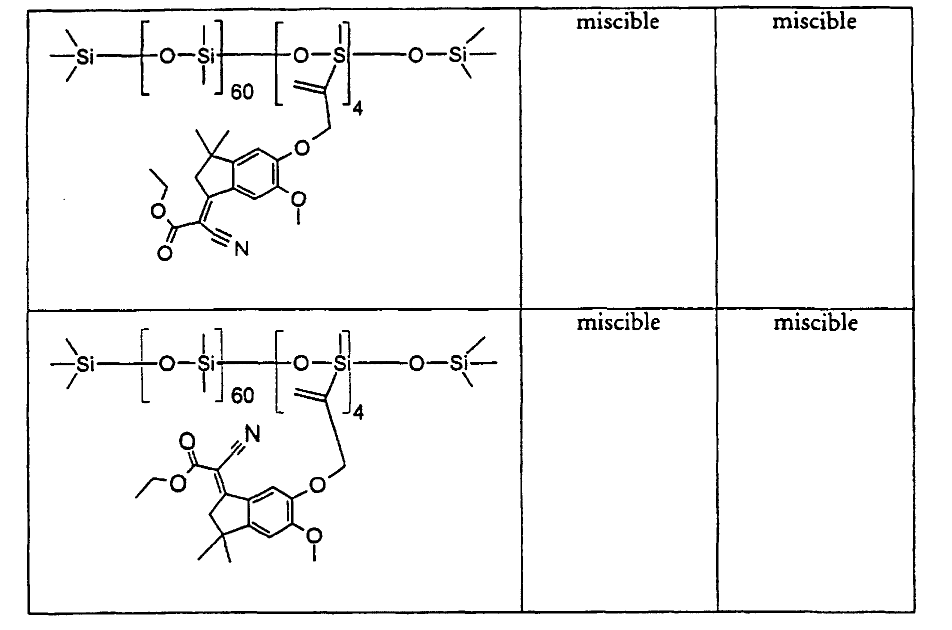

The following Examples 14-16 illustrate light screening agents provided by the present invention.

In these examples the trade names selected have the following significance:
- AMPHISOL DEA:: Diethanolamine cetylphosphate sold under the tradename AMPHISOL DEA by Givaudan Roure S.A, F-95101 Argenteuil-Paris.
- CARBOPOL 934:: Carbomer sold under the tradename CARBOPOL 934 by B.F. Goodrich Company, Brecksville, OH 44141, USA.
- CERAPHYL 375:: Isostearyl neopentanoate sold under the tradename CERAPHYL 375 by ISP Global Technologies Deutschland GmbH, Frechen, Germany.
- CERAPHYL 847:: Octyldodecyl stearoyl stearate sold under the tradename CERAPHYL 847 by ISP.
- CETIOL LC:: Coco-caprylate/caprate sold under the tradename CETIOL LC by Henkel KgA, Düsseldorf, Germany.
- CRODAMOL DA:: Diisopropyladipate sold under the tradename CRODAMOL DA by Croda.
- DERMOL 185:: Isostearyl neopentanoate sold under the tradename DERMOL 185 by Bernel.
- EDETA BD:: Disodium EDTA sold under the tradename EDETA BD by BASF AG, Ludwigshafen, Germany.
- ESTOL GTEH 3609:: Trioctanoin sold under the trade name ESTOL GTEH 3609 by Unichema Chemie GmbH, Emmerich, Germany.
- ESTOL GMM 3650:: Glyceryl Myristate sold under the trade name ESTOL GMM 3650 by Unichema.
- GANEX V-220:: PVP/Eicosene copolymer sold under the tradename GANEX V-220 by ISP.
- NIPAGIN M:: Methylparaben sold under the tradename NIPAGIN M by Nipa Lab. Ltd., Pontypridd Mid Glam, Wales/GB
- PARSOL MCX:: Octyl methoxycinnamate sold under the tradename PARSOL MCX by F. Hoffmann-la Roche Ltd,CH-4070 Basel.
- PARSOL 1789:: 4-t-Butyl-4'-methoxy-dibenzoyl-methane sold under the trade name PARSOL 1789 by Roche.
- PARSOL 5000:: 4-Methylbenzylidene camphor sold under the tradename PARSOL 5000 by Roche.
- PHENONIP:: Phenoxyethanol & Methyl-, Ethyl-, Propyl- & Butyl-paraben sold under the tradename PHENONIP by Nipa.
- T-COTE 031:: Titanium Dioxide & Dimethicone sold under the tradename T-COTE 031 by Sunsmart, Wainscott-NY 11975, USA

### Example 14

Preparation of a O/W broad spectrum sunscreen lotion containing 2% of the product described in Example 2.

| % w/w | Ingredient | Chemical Name/INCI Name |
|---|---|---|
| Part A | | |
| 2.0 | PARSOL MCX | Octyl methoxycinnamate |
| 2.0 | Product of Example 2 | |
| 3.0 | PARSOL 1789 | 4-t-Butyl-4'-methoxy-dibenzoyl-methane |
| 12.0 | CETIOL LC | Coco-caprylate/caprate |
| 4.0 | DERMOL 185 | Isostearyl neopentanoate |
| 0.25 | Diethyleneglycol monostearate | PEG-2-stearate |
| 1.0 | Cetylalcohol | Cetylalcohol |
| 0.25 | MPOB/PPOB | Methyl-propylparabene |
| 0.1 | EDTA BD | EDTA-sodium salt |
| 1.0 | AMPHISOL DEA | Diethanolamine cetylphosphate |

| Part B | | |
|---|---|---|
| 20.0 | Permulene TR-1 (+%) | Acrylate C10-C30 Alkylacrylate |
| 48.6 | Deionized Water | Deionized Water |
| 5.0 | Propyleneglycol | 1,2-Propanediol |
| 0.8 | KOH (10%) | Potassium hydroxyde |

Part A was heated in a reactor to 85°C. Part B was slowly added within 10 min., followed by addition of KOH, cooling and degassing of the emulsion.

### Example 15

Preparation of a O/W anionic broad spectrum sunscreen lotion containing 4% of the product described in Example 8.

| % w/w | Ingredient | Chemical Name/INCI Name |
|---|---|---|
| Part A | | |
| 3.0 | PARSOL MCX | Octyl methoxycinnamate |
| 4.0 | Product of Example 8 | |
| 3.0 | PARSOL 5000 | 4-Methylbenzylidene camphor |
| 4.0 | PARSOL 1789 | 4-t-Butyl-4'-methoxy-dibenzoyl-methane |
| 2.0 | Glyceryl monostearate | Glyceryl stearate |
| 2.0 | Cetyl alcohol extra | Cetyl alcohol |
| 2.0 | GANEX V-220 | PVP/Eicosene copolymer |
| 4.0 | CERAPHYL 375 | Isostearyl neopentanoate |
| 4.0 | CERAPHYL 847 | Octyldodecyl stearoyl stearate |
| 2.0 | AMPHISOL K | Potassium cetylphosphate |
| 0.1 | EDETA BD | Disodium EDTA |
| 0.6 | PHENONIP | Phenoxyethanol & Methyl-, Ethyl-, Propyl- & Butyl-paraben |

| Part B | | |
|---|---|---|
| 11.2 | Deionized Water | Deionized Water |
| 50.0 | CARBOPOL 9341% solution | Carbomer |
| 5.0 | Propyleneglycol | 1,2-Propanediol |
| 0.2 | NIPAGIN M | Methylparaben |
| 3.0 | KOH ( 10%) | Potassium hydroxyde |
| q.s. | Perfume oil | Fragrance |

Part A was heated in a reactor to 85°C. When homogeneous Part B was added, followed by addition of preheated KOH (75°C), cooling and degassing of the emulsion.

### Example 16

Preparation of a O/W broad spectrum sunscreen cream with pigments having low skin penetration quality and containing 4% of the product described in Example 9.

| % w/w | Ingredients | Chemical Name/INCI Name |
|---|---|---|
| Part A | | |
| 8.0 | Polysiloxane A described in | Polysiloxane grafted benzalmalonate UV-B |
| | EP 0709080 A1 | sunscreen |
| 4.0 | Product of Example 9 | |
| 6.0 | T-COTE 031 | Titanium Dioxide & Dimethicone |
| 10.0 | ESTOL GTEH 3609 | Trioctanoin |
| 1.0 | Cetyl Alcohol | Cetyl Alcohol |
| 4.0 | ESTOL GMM 3650 | Glyceryl Myristate |
| 0.05 | Butylated Hydroxytoluene | BHT |
| 0.1 | EDETA BD | Disodium EDTA |
| 0.6 | PHENONIP | Phenoxyethanol & Methylparaben & |
| | | Ethylparaben & Propylparaben & |
| | | Butylparaben |
| 2.0 | AMPHISOL K | Potassium Cetyl Phosphate |

| Part B | | |
|---|---|---|
| 50.8 | Deionized Water | Deionized Water |
| 10.0 | Carbopol 980 1% sol'n | Carbomer 980 |
| 3.0 | Glycerin | Glycerin |
| Part C | | |
| 0.5 | KOH 10 % sol'n | Potassium Hydroxide |
| Part D | | |
| q.s. | Perfume Oil | Fragance |

### Procedure

The ingredients of Part A were heated to 85°C while stirring. After mixing for 30 sec. with a turbine at 8000 t/min. the ingredients of Part B and Part C were added to the homogeneous mixture. The mixture was heated to 75°C, while stirring. After cooling to 40°C the ingredients of Part D were added. The water loss was compensated and the mixture was cooled to room temperature under stirring followed by mixing for 30 sec. with a turbine at 8000 t/min.

## Claims

1. Compounds of the general formula I 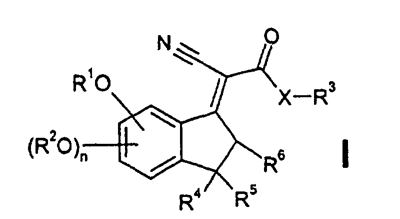 wherein
X signifies O or NH;
R¹ signifies C₁-C₂₀ alkyl, C₂-C₂₀ alkyl in which at least one methylene group is replaced by oxygen, C₃-C₂₀ alkenyl, C₃-C₂₀ alkynyl or a group YS;
R² signifies C₁-C₂₀ alkyl, C₂-C₂₀ alkyl in which at least one methylene group is replaced by oxygen, C₃-C₂₀ alkenyl, C₃-C₂₀ alkynyl or a group YS; or R¹ and R² can combine on adjacent C-atoms to form a dioxomethylene ring;
R³ signifies C₁-C₂₀ alkyl, C₂-C₂₀ alkyl in which at least one methylene group is replaced by oxygen, C₃-C₂₀ alkenyl, C₃-C₂₀ alkynyl or a group YS;
R⁴,R⁵,R⁶ each independently signify H or C₁-C₂₀ alkyl;
n signifies 0, 1 or 2;
Y signifies a linker group;
S signifies a silane-, an oligosiloxane- or a polysiloxane-moiety;
with the proviso that at least one of the residues R¹, R² or R³ signifies YS.

2. Compounds according to claim 1, wherein R² signifies C₁-C₂₀ alkyl, C₂-C₂₀ alkyl in which at least one methylene group is replaced by oxygen, C₃-C₂₀ alkenyl, C₃-C₂₀ alkynyl; or R¹ and R² can combine on adjacent C-atoms to form a dioxomethylene ring.

3. Compounds according to claim 1 or 2, wherein the linker group signifies a C₃-C₁₂ divalent alkylene or alkenylene chain which links the silane, oligosiloxane or polysiloxane moiety to the UV absorbing chromophoric residue.

4. Compounds according to claim 3, wherein the linker group signifies 3-propylene, 2-propylene, 2-methyl-3-propylene, 3-butylene, 4-butylene, 4-pentylene, 5-pentylene, 6-hexylene, 2-propen-2-ylene, 2-propen-3-ylene, 3-buten-3-ylene, 3-buten-4-ylene, 4-penten-4-ylene, 4-penten-5-ylene, (3-methyl)-penta-2,4-dien-4 or 5-ylene, 11-dodecen-11-ylene, 2-ethyloxy-eth-2-ylene, 4-butyloxy-eth-2-ylene or 3,6-dioxa-8-octylene.

5. Compounds according to any one of claims 1-4, wherein S signifies a group of the general formula -SiR⁷R⁸R⁹ wherein R⁷, R⁸ and R⁹ each independently signify C₁-C₆ alkyl, C₁-C₆ alkoxy or phenyl.

6. Compounds according to claim 5, wherein S signifies trimethylsilane, triethylsilane, tripropylsilane, triisopropylsilane, dimethyl tert.butylsilane, dimethyl thexylsilane, triphenylsilane, dimethylphenylsilane.

7. Compounds according to any one of claims 1-4, wherein S signifies a group of the general formula -SiR¹⁰ₘ(OSiR¹⁰₃)ₙ with m = 0, 1 or 2; n = 3, 2 or 1 and m+n = 3; or groups of the general formula IIa or IIb 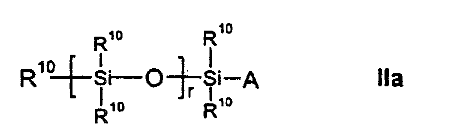 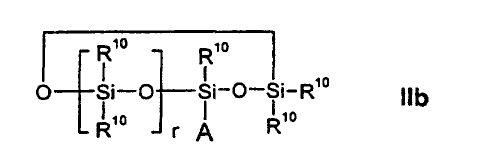 wherein
A signifies a bond to the linker Y;
R¹⁰ signifies C₁-C₆ alkyl or phenyl;
r signifies 1 to 9.

8. Compounds according to any one of claims 1-4, wherein S signifies a polysiloxane group of the general formula IIIa or IIIb, 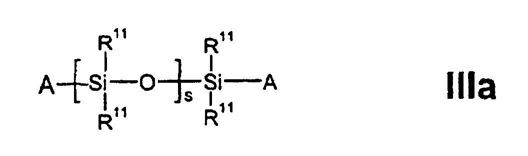 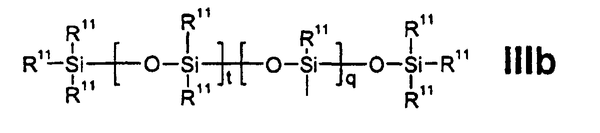 wherein
A is a bond to the linker Y;
R¹¹ signifies C₁-C₆ alkyl or phenyl;
s has a value of from 4 to 250;
t has a value of from 5 to 250;
q has a value of from 1 to 30.

9. Compounds according to any one of claims 1-8, wherein R¹ and R² signify C₁-C₆ alkyl, preferably C₁-C₄ alkyl, more preferably methyl, or a group YS.

10. Compounds according to any one of claims 1-9, wherein R³ signifies C₁-C₆ alkyl, preferably C₁-C₄ alkyl, more preferably ethyl, or a group YS.

11. Compounds according to any one of claims 1-10, wherein R⁴ and R⁵ signify C₁-C₆ alkyl, preferably C₁-C₄ alkyl, more preferably methyl.

12. Compounds according to any one of claims 1-11, wherein R⁶ signifies C₁-C₆ alkyl, preferably C₁-C₄ alkyl, more preferably methyl, or hydrogen.

13. Compounds according to any one of claims 7 and 9-12, wherein R¹⁰ signifies C₁-C₆ alkyl, preferably C₁-C₄ alkyl, more preferably methyl.

14. Compounds according to any one of claims 7 and 9-13, wherein r is 1 to 3.

15. Compounds according to any one of claims 8-12, wherein R¹¹ signifies C₁-C₆ alkyl, preferably C₁-C₄ alkyl, more preferably methyl.

16. Compounds according to any one of claims 8-12 and 15, wherein s is 5 to 150.

17. Compounds according to any one of claims 8-12, 15 and 16, wherein t is 5 to 150, preferably a statistical mean value of about 60.

18. Compounds according to any on of claims 8-12 and 15-17, wherein q is 2 to 10, preferably a statistical mean value of about 4.

19. Compounds according to any one of claims 1-18, wherein n is 0 or 1.

20. Light screening composition comprising a compound according to any one of claims 1-19.

21. Light screening composition according to claim 20, **characterized in that** it contains, in addition, common UV-A and/or UV-B screening agents.

22. The use of a compound according to any one of claims 1-19 as absorber for the ultraviolet light.

## Patentansprüche

1. Verbindungen der allgemeinen Formel I 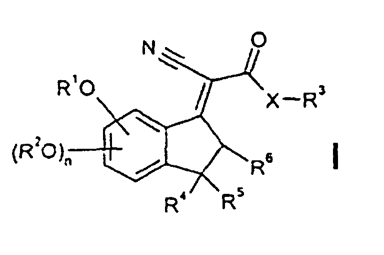 worin
X O oder NH bedeutet;
R¹ C₁-C₂₀-Alkyl, C₂-C₂₀-Alkyl, bei dem mindestens eine Methylengruppe durch Sauerstoff ersetzt ist, C₃-C₂₀-Alkenyl, C₃-C₂₀-Alkinyl oder eine Gruppe YS bedeutet;
R² C₁-C₂₀-Alkyl, C₂-C₂₀-Alkyl, bei dem mindestens eine Methylengruppe durch Sauerstoff ersetzt ist, C₃-C₂₀-Alkenyl, C₃-C₂₀-Alkinyl oder eine Gruppe YS bedeutet oder R¹ und R² zusammen mit benachbarten C-Atomen einen Dioxomethylenring bilden können;
R³ C₁-C₂₀-Alkyl, C₂-C₂₀-Alkyl, bei dem mindestens eine Methylengruppe durch Sauerstoff ersetzt ist, C₃-C₂₀-Alkenyl, C₃-C₂₀-Alkinyl oder eine Gruppe YS bedeutet;
R⁴, R⁵, R⁶ jeweils unabhängig H oder C₁-C₂₀-Alkyl bedeuten;
n 0, 1 oder 2 bedeutet;
Y eine Linkergruppe bedeutet;
S einen Silan-, Oligosiloxan- oder Polysiloxananteil bedeutet
mit dem Vorbehalt, dass mindestens einer der Reste R¹, R² oder R³ YS bedeutet.

2. Verbindungen nach Anspruch 1, wobei R² C₁-C₂₀-Alkyl, C₂-C₂₀-Alkyl, bei dem mindestens eine Methylengruppe durch Sauerstoff ersetzt ist, C₃-C₂₀-Alkenyl, C₃-C₂₀-Alkinyl bedeutet oder R¹ und R² zusammen mit benachbarten C-Atomen einen Dioxomethylenring bilden.

3. Verbindungen nach Anspruch 1 oder Anspruch 2, wobei die Linkergruppe eine divalente C₃-C₁₂-Alkylen- oder Alkenylenkette bedeutet, die den Silan-, Oligosiloxan- oder Polysiloxananteil mit dem UV-absorbierenden chromophoren Rest verbindet.

4. Verbindungen nach Anspruch 3, wobei die Linkergruppe 3-Propylen, 2-Propylen, 2-Methyl-3-propylen, 3-Butylen, 4-Butylen, 4-Pentylen, 5-Pentylen, 6-Hexylen, 2-Propen-2-ylen, 2-Propen-3-ylen, 3-Buten-3-ylen, 3-Buten-4-ylen, 4-Penten-4-ylen, 4-Penten-5-ylen, (3-Methyl)penta-2,4-dien-4- oder -5-ylen, 11-Dodecen-11-ylen, 2-Ethyloxyeth-2-ylen, 4-Butyloxy-eth-2-ylen oder 3,6-Dioxa-8-octylen bedeutet.

5. Verbindungen nach einem der Ansprüche 1 bis 4, wobei S eine Gruppe der allgemeinen Formel -SiR ⁷R⁸R⁹ bedeutet, worin R⁷, R⁸ und R⁹ jeweils unabhängig C₁-C₆-Alkyl, C₁-C₆-Alkoxy oder Phenyl bedeuten.

6. Verbindungen nach Anspruch 5, wobei S Trimethylsilan, Triethylsilan, Tripropylsilan, Triisopropylsilan, Dimethyl-tert.-butylsilan, Dimethylthexylsilan, Triphenylsilan, Dimethylphenylsilan bedeutet.

7. Verbindungen nach einem der Ansprüche 1 bis 4, wobei S eine Gruppe der allgemeinen Formel -SiR¹⁰ₘ(OSiR¹⁰₃)ₙ bedeutet, mit m = 0, 1 oder 2; n = 3, 2 oder 1 und m+n = 3 oder Gruppen der allgemeinen Formel IIa oder IIb 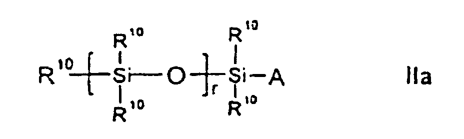 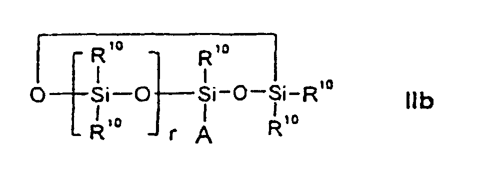 worin
A eine Bindung zu dem Linker Y bedeutet;
R¹⁰ C₁-C₆-Alkyl oder Phenyl bedeutet;
r 1 bis 9 bedeutet.

8. Verbindungen nach einem der Ansprüche 1 bis 4, worin S eine Polysiloxangruppe der allgemeinen Formel IIIa oder IIIb bedeutet 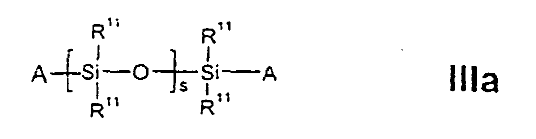 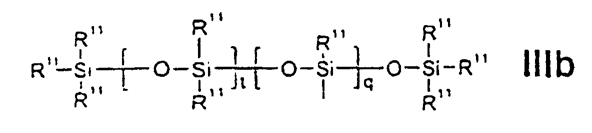 worin
A eine Bindung zu dem Linker Y ist;
R¹¹ C₁-C₆-Alkyl oder Phenyl bedeutet;
s einen Wert von 4 bis 250 hat;
t einen Wert von 5 bis 250 hat;
q einen Wert von 1 bis 30 hat.

9. Verbindungen nach einem der Ansprüche 1 bis 8, wobei R¹ und R² C₁-C₆-Alkyl, bevorzugt C₁-C₄-Alkyl, bevorzugter Methyl, oder eine Gruppe YS bedeuten.

10. Verbindungen nach einem der Ansprüche 1 bis 9, wobei R³ C₁-C₆-Alkyl, bevorzugt C₁-C₄-Alkyl, bevorzugter Ethyl, oder eine Gruppe YS bedeutet.

11. Verbindungen nach einem der Ansprüche 1 bis 10, wobei R⁴ und R⁵ C₁-C₆-Alkyl, bevorzugt C₁-C₄-Alkyl, bevorzugter Methyl bedeuten.

12. Verbindungen nach einem der Ansprüche 1 bis 11, wobei R⁶ C₁-C₆-Alkyl, bevorzugt C₁-C₄-Alkyl, bevorzugter Methyl, oder Wasserstoff bedeutet.

13. Verbindungen nach einem der Ansprüche 7 und 9 bis 12, wobei R¹⁰ C₁-C₆-Alkyl, bevorzugt C₁-C₄-Alkyl, bevorzugter Methyl bedeutet.

14. Verbindungen nach einem der Ansprüche 7 und 9 bis 13, wobei r 1 bis 3 ist.

15. Verbindungen nach einem der Ansprüche 8 bis 12, wobei R¹¹ C₁-C₆-Alkyl, bevorzugt C₁-C₄-Alkyl, bevorzugter Methyl bedeutet.

16. Verbindungen nach einem der Ansprüche 8 bis 12 und 15, wobei s 5 bis 150 ist.

17. Verbindungen nach einem der Ansprüche 8 bis 12, 15 und 16, wobei t 5 bis 150, bevorzugt ein statistischer Mittelwert von etwa 60 ist.

18. Verbindungen nach einem der Ansprüche 8 bis 12 und 15 bis 17, wobei q 2 bis 10, bevorzugt ein statistischer Mittelwert von etwa 4 ist.

19. Verbindungen nach einem der Ansprüche 1 bis 18, wobei n 0 oder 1 ist.

20. Lichtschutzzusammensetzung enthaltend eine Verbindung nach einem der Ansprüche 1 bis 19.

21. Lichtschutzzusammensetzung nach Anspruch 20, **dadurch gekennzeichnet, dass** sie zusätzlich übliche UV-A- und/oder UV-B-Schutzmittel enthält.

22. verwendung einer Verbindung nach einem der Ansprüche 1 bis 19 als Absorber für Ultraviolettlicht.

## Revendications

1. Composés de formule générale 1 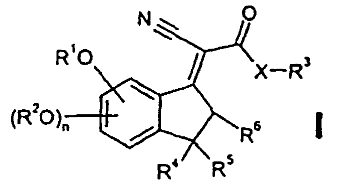 dans laquelle
X représente O ou NH ;
R' représente un alkyle en C₁-C₂₀, un alkyle en C₂-C₂₀ dont un groupe méthylène au moins est remplacé par un oxygène, un alcényle en C₃-C₂₀, un alcynyle en C₃-C₂₀ ou un groupe YS ;
R² représente un alkyle en C₁-C₂₀, un alkyle en C₂-C₂₀ dont un groupe méthylène au moins est remplacé par un oxygène, un alcényle en C₃-C₂₀, un alcynyle en C₃-C₂₀ ou un groupe YS ; ou bien R¹ et R² peuvent, combinés avec les atomes de carbone qui leur sont adjacents, former un cycle dioxométhylène ;
R³ représente un alkyle en C₁-C₂₀, un alkyle en C₂-C₂₀ dont un groupe méthylène au moins est remplacé par un oxygène, un alcényle en C₃-C₂₀, un alcynyle en C₃-C₂₀ ou un groupe YS ;
R⁴, R⁵, R⁶ représentent chacun de façon indépendante H ou un alkyle en C₁-C₂₀;
n représente 0, 1 ou 2 ;
Y représente un groupe lieur ;
S représente un fragment silane, oligosiloxane ou polysiloxane ;
sous réserve qu'au moins un des restes R¹, R² ou R³ représente YS.

2. Composés selon la revendication 1, où R² représente un alkyle en C₁-C₂₀, un alkyle en C₂-C₂₀ dont un groupe méthylène au moins est remplacé par un oxygène, un alcényle en C₃-C₂₀, un alcynyle en C₃-C₂₀ ; ou bien R¹ et R² peuvent, combinés avec les atomes de carbone qui leur sont adjacents, former un cycle dioxométhylène.

3. Composés selon la revendication 1 ou 2, où le groupe lieur est une chaîne alkylène ou alcénylène divalente en C₃-C₁₂ qui relie le fragment silane, oligosiloxane ou polysiloxane au reste chromophore absorbant les UV.

4. Composés selon la revendication 3, où le groupe lieur représente le 3-propylène, le 2-propylène, le 2-méthyl-3-propylène, le 3-butylène, le 4-butylène, le 4-pentylène, le 5-pentylène, le 6-hexylène, le 2-propén-2-ylène, le 2-propén-3-ylène, le 3-butén-3-ylène, le 3-butén-4-ylène, le 4-pentén-4-ylène, le 4-pentén-5-ylène, le (3-méthyl)penta-2,4-dién-4- ou -5-ylène, le 11-dodécén-11-ylène, le 2-éthyloxyéth-2-ylène, le 4-butyloxyéth-2-ylène ou le 3,6-dioxa-8-octylène.

5. Composés selon l'une quelconque des revendications 1 à 4, où S représente un groupe de formule générale -SiR⁷R⁸R⁹, dans lequel R⁷, R⁸ et R⁹ représentent, chacun de façon indépendante, un alkyle en C₁-C₆, un alcoxy en C₁-C₆ ou un phényle.

6. Composés selon la revendication 5, où S signifie triméthylsilane, triéthylsilane, tripropylsilane, triisopropylsilane, diméthyl-tert-butylsilane, diméthylhexylsilane, triphénylsilane, diméthylphénylsilane.

7. Composés selon l'une quelconque des revendications 1 à 4, où S représente un groupe de formule générale -SiR¹⁰ₘ(OSiR¹⁰₃)ₙ dans lequel m = 0, 1 ou 2 ; n = 3, 2 ou 1 et m + n = 3 ; ou des groupes de formule générale IIa ou IIb 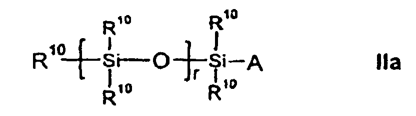 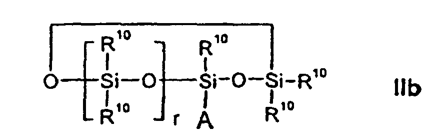 où
A représente une liaison au lieur Y ;
R¹⁰ représente un alkyle en C₁-C₆ ou un phényle ;
r a une valeur de 1 à 9.

8. Composés selon l'une quelconque des revendications 1 à 4, où S représente un groupe polysiloxane de formule générale. IIIa ou IIIb 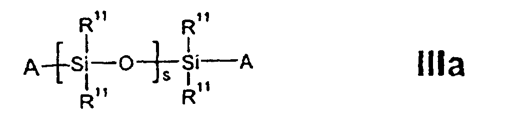 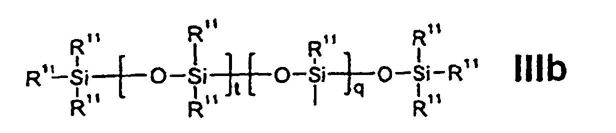 où
A représente une liaison au lieur Y ;
R¹¹ représente un alkyle en C₁-C₆ ou un phényle;
s a une valeur de 4 à 250 ;
t a une valeur de 5 à 250 ;
q a une valeur de 1 à 30.

9. Composés selon l'une quelconque des revendications 1 à 8, où R¹ et R² représentent un alkyle en C₁-C₆, de préférence un alkyle en C₁-C₄, plus préférablement un méthyle, ou un groupe YS.

10. Composés selon l'une quelconque des revendications 1 à 9, où R³ représente un alkyle en C₁-C₆, de préférence un alkyle en C₁-C₄, plus préférablement un éthyle, ou un groupe YS.

11. Composés selon l'une quelconque des revendications 1 à 10, où R⁴ et R⁵ représentent un alkyle en C₁-C₆, de préférence un alkyle en C₁-C₄, plus préférablement un méthyle.

12. Composés selon l'une quelconque des revendications 1 à 11, où R⁶ représente un alkyle en C₁-C₆, de préférence un alkyle en C₁-C₄, plus préférablement un méthyle, ou un hydrogène.

13. Composés selon l'une quelconque des revendications 7 et 9 à 12, où R¹⁰ représente un alkyle en C₁-C₆, de préférence un alkyle en C₁-C₄, plus préférablement un méthyle.

14. Composés selon l'une quelconque des revendications 7 et 9 à 13, où r a une valeur de 1 à 3.

15. Composés selon l'une quelconque des revendications 8 à 12, où R¹¹ représente un alkyle en C₁-C₆, de préférence un alkyle en C₁-C₄, plus préférablement un méthyle.

16. Composés selon l'une quelconque des revendications 8 à 12 et 15, où s a une valeur de 5 à 150.

17. Composés selon l'une quelconque des revendications 8 à 12, 15 et 16, où t a une valeur de 5 à 150, de préférence a une valeur moyenne statistique d'environ 60.

18. Composés selon l'une quelconque des revendications 8 à 12 et 15 à 17, où q a une valeur de 2 à 10, de préférence a une valeur moyenne statistique d'environ 4.

19. Composés selon l'une quelconque des revendications 1 à 18, où n est égal à 0 ou 1.

20. Composition formant écran à la lumière comprenant un composé selon l'une quelconque des revendications 1 à 19.

21. Composition formant écran à la lumière selon la revendication 20, **caractérisée en ce qu'**elle contient, en outre, des agents classiques filtrant les UV-A et/ou les UV-B.

22. Utilisation d'un composé selon l'une quelconque des revendications 1 à 19 en tant qu'absorbant pour la lumière ultraviolette.
